# EUROPEAN PATENT SPECIFICATION

(11) **EP 3 103 799 B1**
(45) Date of publication and mention of the grant of the patent: **06.06.2018**
(21) Application number: 16171536.2
(22) Date of filing: 06.06.1995
(51) Int. Cl.: C07D 239/94, A61K 31/517

(54) **QUINAZOLINE DERIVATIVES**
CHINAZOLINDERIVATE
DERIVES DE QUINAZOLINE

(30) Priority: 30.03.1995 US 413300
(43) Date of publication of application: 14.12.2016
(62) Divisional of application: 09174228.8
(73) Proprietor: OSI Pharmaceuticals, LLC, Northbrook, IL 60062 (US); Pfizer Products Inc., Groton, CT 06340 (US)
(72) Inventor: SCHNUR, Rodney Caughren, Noank, CT Connecticut 06340 (US); ARNOLD, Lee Daniel, Westborough, MA Massachusetts 01581 (US)
(74) Representative: Crump, Julian Richard John

(56) References cited:
- EP-A- 0 635 498
- WO-A-95/15758

## Description

### Background of the Invention

This invention relates to 4-(substitutedphenylamino)quinazoline derivatives which are useful in the treatment of hyperproliferative diseases, such as cancers, in mammals.

Many of the current treatment regimes for cancer utilize compounds which inhibit DNA synthesis. Such compounds are toxic to cells generally but their toxic effect on the rapidly dividing tumor cells can be beneficial. Alternative approaches to anti-cancer agents which act by mechanisms other than the inhibition of DNA synthesis have been explored in order to enhance the selectivity of action against cancer cells.

It is known that a cell may become cancerous by virtue of the transformation of a portion of its DNA into an oncogene (i.e. a gene which, on activation, leads to the formation of malignant tumor cells). Many oncogenes encode proteins which are aberrant tyrosine kinases capable of causing cell transformation. Alternatively, the overexpression of a normal proto-oncogenic tyrosine kinase may also result in proliferative disorders, sometimes resulting in a malignant phenotype.

Receptor tyrosine kinases are large enzymes which span the cell membrane and possess an extracellular binding domain for growth factors such as epidermal growth factor, a transmembrane domain, and an intracellular portion which functions as a kinase to phosphorylate specific tyrosine residues in proteins and hence to influence cell proliferation. It is known that such kinases are frequently aberrantly expressed in common human cancers such as breast cancer, gastrointestinal cancer such as colon, rectal or stomach cancer, leukemia, and ovarian, bronchial or pancreatic cancer. It has also been shown that epidermal growth factor receptor (EGFR) which possesses tyrosine kinase activity is mutated and/or overexpressed in many human cancers such as brain, lung, squamous cell, bladder, gastric, breast, head and neck, oesophageal, gynecological and thyroid tumors.

Accordingly, it has been recognized that inhibitors of receptor tyrosine kinases are useful as a selective inhibitors of the growth of mammalian cancer cells. For example, erbstatin, a tyrosine kinase inhibitor selectively attenuates the growth in athymic nude mice of a transplanted human mammary carcinoma which expresses epidermal growth factor receptor tyrosine kinase (EGFR) but is without effect on the growth of another carcinoma which does not express the EGF receptor.

Various other compounds, such as styrene derivatives, have also been shown to possess tyrosine kinase inhibitory properties. More recently five European patent publications, namely EP 0 566 226 A1, EP 0 602 851 A1, EP 0 635 507 A1, EP 0 635 498 A1 and EP 0 520 722 A1 have disclosed that certain quinazoline derivatives possess anti-cancer properties which result from their tyrosine kinase inhibitory properties. Also PCT publication WO 92/20642 discloses bis-mono and bicyclic aryl and heteroaryl compounds as tyrosine kinase inhibitors.

Although the anti-cancer compounds described above make a significant contribution to the art there is a continuing search in this field of art for improved anticancer pharmaceuticals.

### Summary of the Invention

In a first aspect this invention is directed to a composition for use as a medicament for treating hyperliferative diseases comprising a combination of a 4-(substituted phenylamino) quinazoline derivative of the formula 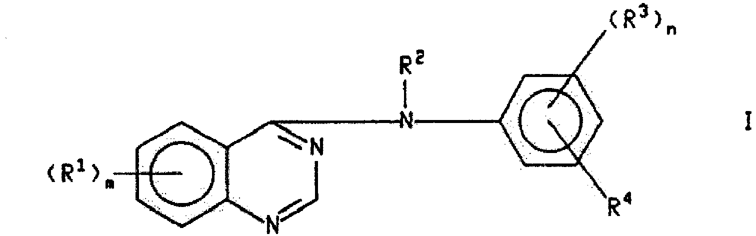 or a pharmaceutically acceptable salt thereof, wherein
m is 2;
each R¹ is 2-methoxyethoxy;
R² is hydrogen;
n is 1 and each R³ is hydrogen; and
R⁴ is R¹¹-ethynyl wherein R¹¹ is hydrogen; and one or more additional antitumor substances.

In particular therefore, the invention relates to [6,7-bis(2-methoxyethoxy)quinazolin-4-yl]-(3-elhynylphenyl)amine.

In a second aspect this invention is directed to the 4-(substitutedphenylamine) quinazoline derivative as defined above, or a pharmaceutically acceptable salt thereof, in combination with one or more additional antitumor substances for treating hyperproliferative diseases.

In a third aspect this invention is directed to a pharmaceutical combination comprising: the 4-(substitutedphenylamine)quinazoline derivative as defined above, or a pharmaceutically acceptable salt thereof; and one or more additional antitumor substances.

As used herein, the expression "reaction-inert solvent" refers to a solvent which does not interact with starting materials, reagents, intermediates or products in a manner which adversely affects the yield of the desired product.

Other features and advantages will be apparent from the specification and claims which describe the invention.

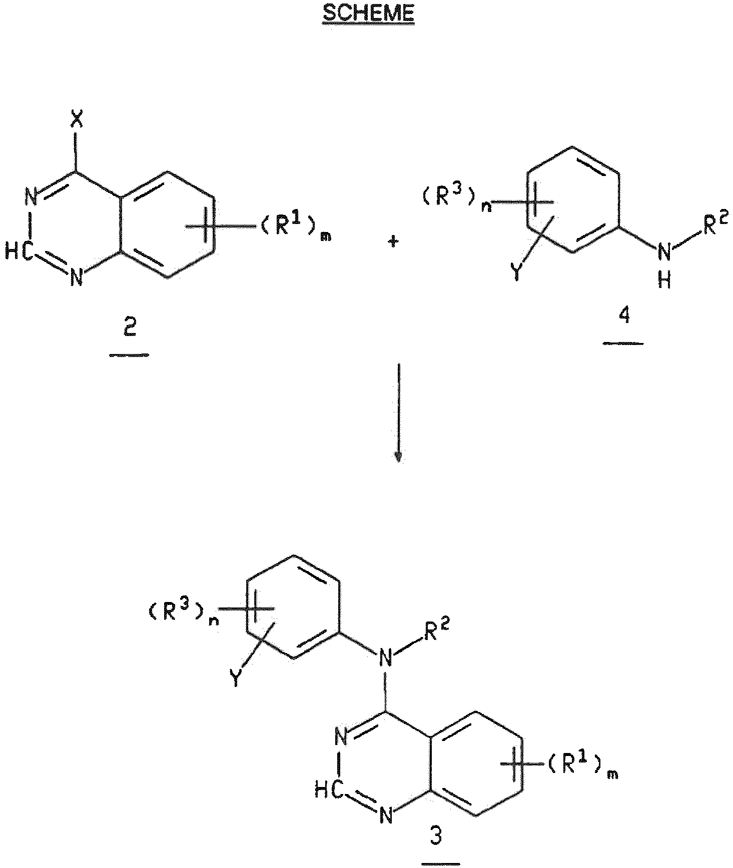

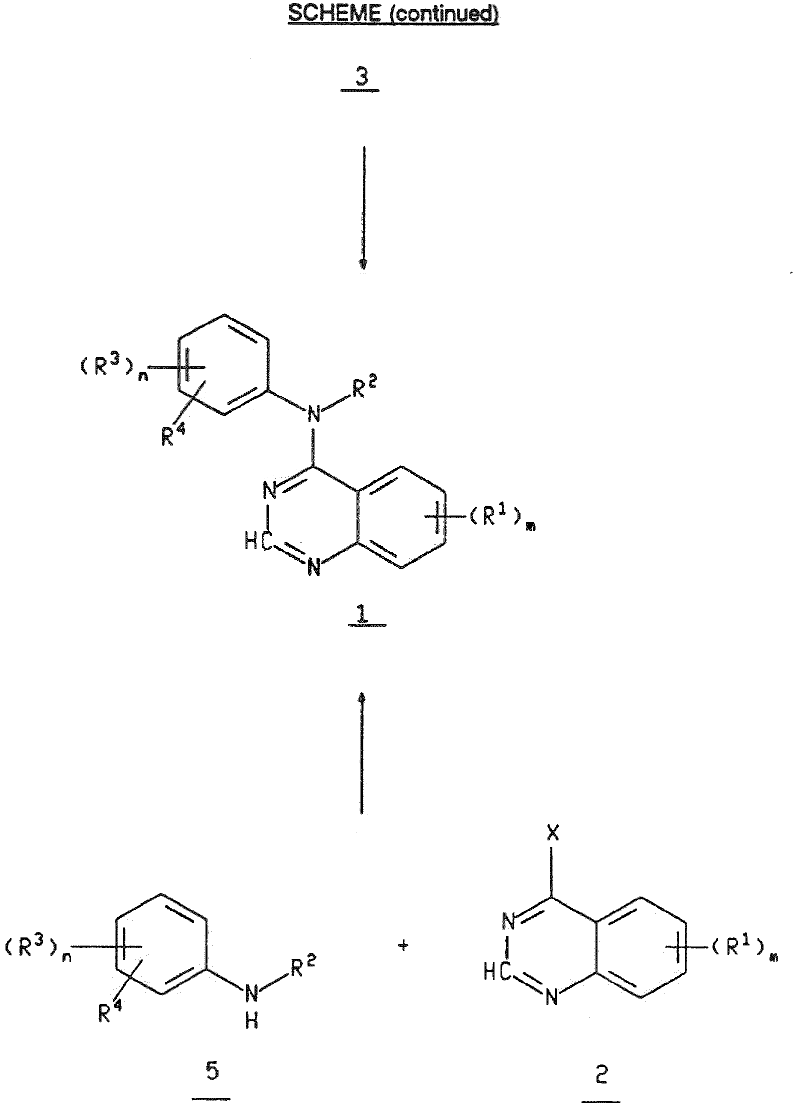

### Detailed Description of the Invention

The Formula I compound and pharmaceutically acceptable salts thereof (hereafter the active compounds) may be prepared by any process known to be applicable to the preparation of chemically-related compounds.

In general the active compound may be made from the appropriately substituted quinazoline using the appropriately substituted amine.

As shown in the Scheme the appropriate 4-substituted quinazoline 2 wherein X is a suitable displaceable leaving group such as halo, aryloxy, alkylsulfinyl, alkylsulfonyl such as trifluoromethanesulfonyloxy, arylsulfinyl, arylsulfonyl, siloxy, cyano, pyrazolo, triazolo or tetrazolo, preferably a 4-chloroquinazoline, is reacted with the appropriate amine or amine hydrochloride 4 or 5, wherein R⁴ is as described above and Y is Br, I, or trifluoromethane-sulfonyloxy in a solvent such as a (C₁-C₆)alcohol, dimethylformamide (DMF), N-methylpyrrolidin-2-one, chloroform, acetonitrile, tetrahydrofuran (THF), 1-4 dioxane, pyridine or other aprotic solvent. The reaction may be effected in the presence of a base, preferably an alkali or alkaline earth metal carbonate or hydroxide or a tertiary amine base, such as pyridine, 2,6-lutidine, collidine, N-methyl-morpholine, triethylamine, 4-dimethylamino-pyridine or N,N-dimethylaniline. These bases are hereinafter refered to as suitable bases. The reaction mixture is maintained at a temperature from about ambient to about the reflux temperature of the solvent, preferably from about 35°C to about reflux, until substantially no remaining 4-haloquinazoline can be detected, typically about 2 to about 24 hours. Preferably, the reaction is performed under an inert atmosphere such as dry nitrogen.

Generally the reactants are combined stoichiometrically. When an amine base is used for those compounds where a salt (typically the HCl salt) of an amine 4 or 5 is used, it is preferable to use excess amine base, generally an extra equivalent of amine base. (Alternatively, if an amine base is not used an excess of the amine 4 or 5 may be used).

For compounds where a sterically hindered amine 4 is used it is preferable to use t-butyl alcohol or a polar aprotic solvent such as DMF or N-methylpyrrolidin-2-one as the solvent.

Alternatively, a 4-substituted quinazoline 2 wherein X is hydroxyl or oxo (and the 2- nitrogen is hydrogenated) is reacted with carbon tetrachloride and an optionally substituted triarylphosphine which is optionally supported on an inert polymer (e.g. triphenylphosphine, polymer supported, Aldrich Cat. No. 36,645-5, which is a 2% divinylbenzene cross-linked polystyrene containing 3 mmol phosphorous per gram resin) in a solvent such as carbon tetrachloride, chloroform, dichloroethane, tetrahydrofuran, acetonitrile or other aprotic solvent or mixtures thereof. The reaction mixture is maintained at a temperature from about ambient to reflux, preferably from about 35°C to reflux, for 2 to 24 hours. This mixture is reacted with the appropriate amine or amine hydrochloride 4 or 5 either directly or after removal of solvent, for example by vacuum evaporation, and addition of a suitable alternative solvent such as a (C₁-C₅) alcohol, DMF, N-methylpyrrolidin-2-one, pyridine or 1-4 dioxane. Then, the reaction mixture is maintained at a temperature from about ambient to the reflux temperature of the solvent preferably from about 35°C to about reflux, until substantially complete formation of product Is acheived, typically from about 2 to about 24 hours. Preferably the reaction Is performed under an inert atmosphere such as dry nitrogen.

When compound 4, wherein Y is Br, I, or trifluoromethanesulfonyloxy, is used as starting material in the reaction with quinazoline 2, a compound of formula 3 is formed wherein R¹, R², R³, and Y are as described above. Compound 3 is converted to compounds of formula 1 wherein R⁴ is R¹¹ethynyl, and R¹¹ is as defined above, by reaction with a suitable palladium reagent such as tetrakis(triphenylphosphine)palladium or bis(triphenylphosphine)palladium dichloride In the presence of a suitable Lewis acid such as cuprous chloride and a suitable alkyne such as trimethylsilylacetylene, propargyl alcohol or 3-(N,N-dimethylamino)-propyne in a solvent such as diethylamine or triethylamine. Compounds 3, wherein Y is NH₂, may be converted to compounds 1 wherein R⁴ is azide by treatment of compound 3 with a diazotizing agent, such as an acid and a nitrite (e.g., acetic acid and NaNO₂) followed by treatment of the resulting product with an azide, such as NaN₃.

The reduction may conveniently be carried out by any of the many procedures known for such transformations. The reduction may be carried out, for example, by hydrogenation of the nitro compound in a reaction-inert solvent in the presence of a suitable metal catalyst such as palladium, platinum or nickel. A further suitable reducing agent is, for example, an activated metal such as activated iron (produced by washing iron powder with a dilute solution of an acid such as hydrochloric acid). Thus, for example, the reduction may be carried out by heating a mixture of the nitro compound and the activated metal with concentrated hydrochloric acid in a solvent such as a mixture of water and an alcohol, for example, methanol or ethanol, to a temperature in the range, for example, 50 to 150°C, conveniently at or near 70°C. Another suitable class of reducing agents are the alkali metal dithionites, such as sodium dithionite, which may be used in (C1-C₄)alkanoic acids, (C₁-C₆)alkanols, water or mixtures thereof.

The compound of Formula I wherein R¹ is substituted (C₁-C₄)alkoxy is prepared by the alkylation, preferably in the presence of a suitable base, of a corresponding compound wherein R¹ is hydroxy. Suitable alkylating agents include alkyl or substituted alkyl halides, for example, an optionally substituted (C₁-C₄)alkyl chloride, bromide or iodide, in the presence of a suitable base in a reaction-inert solvent and at a temperature In the range of about 10 to 140°C, conveniently at or near ambient temperature.

The starting materials for the above described reaction schemes (e.g., amines and quinazolines) are readily available or can be easily synthesized by those skilled in the art using conventional methods of organic synthesis. For example, the preparation of 2,3-dihydro-1,4-benzoxazine derivatives are described in R. C. Elderfield, W.H. Todd, S. Gerber, Ch. 12 in "Heterocyclic Compounds", Vol. 6, R. C. Elderfield ed., John Wiley and Sons, Inc., N.Y., 1957. Substituted 2,3-dihydro-benzothiazinyl compounds are described by R.C. Elderfield and E.E. Harris In Ch. 13 of Volume 6 of the Elderfield "Heterocyclic Compounds" book.

The Formula I quinazoline can exist in solvated, as well as unsolvated forms, such as the hydrated forms. It is to be understood that the invention encompasses all such solvated, as well as unsolvated forms, which possess activity against hyperproliferative diseases.

A suitable pharmaceutically-acceptable salt of a compound of formula I is, for example, an acid-addition salt of a corresponding compound which is sufficiently basic, e.g., an acid-addition salt with, for example, an inorganic or organic acid such as hydrochloric, hydrobromic, sulphuric, phosphoric, methanesulfonic, benzenesulfonic, trifluoroacetic, citric, lactic or maleic acid. A suitable pharmaceutically-acceptable base-addition salt of a compound of formula I which is acidic is an alkali metal salt, for example, a lithium, sodium or potassium salt; an alkaline earth metal salt, for example, a calcium or magnesium salt; an ammonium salt; or a salt with an organic base which affords a physiologically-acceptable cation for example a salt with methylamine, dimethylamine, trimethylamine, piperidine, morpholine or tris-(2-hydroxyethyl)amine. All such salts are within the scope of this invention and they can be prepared by conventional methods. For example, they can be prepared simply by contacting the acidic and basic entities, usually in a stoichiometric ratio, in either an aqueous, nonaqueous or partially aqueous medium, as appropriate. The salts are recovered by filtration; by precipitation with a non-solvent, preferably an etheral or hydrocarbon solvent, followed by filtration and by evaporation of a solvent, or, in the case of aqueous solutions, by lyophilization.

The active compound of this invention is a potent inhibitor of the erbB family of oncogenic and protooncogenic protein tyrosine kinases such as epidermal growth factor receptor (EGFR), erbB2, HER3, or HER4 and thus is adapted to therapeutic use as antiproliferative agents (e.g., anticancer) in mammals, particularly humans. In particular, the compound of this invention is a therapeutant or prophylactic for the treatment of a variety of human tumors (renal, liver, kidney, bladder, breast, gastric, ovarian, colorectal, prostate, pancreatic, lung, vulval, thyroid, hepatic carcinomas, sarcomas, glioblastomas, various head and neck tumors), and other hyperplastic conditions such as benign hyperplasia of the skin (e.g., psoriasis) or prostate (e.g., BPH). It is, in addition, expected that a quinazoline of the present invention may possess activity against a range of leukemias and lymphoid malignancies.

The active compound may also be expected to be useful in the treatment of additional disorders In which aberrant expression ligand/receptor interactions, activation or signalling events related to various protein tyrosine kinases, whose activity is inhibited by the agent of Formula I, are involved.

Such disorders may include those of neuronal, glial, astrocytal, hypothalamic, and other glandular, macrophagal, epithelial, stromal, and blastocoelic nature in which aberrant function, expression, activation or signalling of the erbB tyrosine kinases may be involved. In addition, compound of Formula I may have therapeutic utility in inflammatory, angiogenic and immunologic disorders involving both identified and as yet unidentified tyrosine kinases which are inhibited by the compound of Formula I.

The in vitro activity of the active compound in inhibiting the receptor tyrosine kinase (and thus subsequent proliferative response, e.g., cancer) may be determined by the procedure detailed below.

Activity of the active compound, in vitro, can be determined by the amount of inhibition of the phosphorylation of an exogenous substrate (e.g., Lys, - Gastrin or polyGluTyr (4:1) random copolymer (I. Posner et. al., J. Biol. Chem. 267 (29), 20638-47 (1992)) on tyrosine by epidermal growth factor receptor kinase by a test compound relative to a control. Affinity purified, soluble human EGF receptor (96 ng) is obtained according to the procedure in G. N. Gill, W. Weber, Methods in Enzymology 146, 82-88 (1987) from A431 cells (American Type Culture Collection, Rockville, MD) and preincubated in a microfuge tube with EGF (2*µ*g/ml) in phosphorylation buffer + vanadate (PBV: 60 mM HEPES, pH 7.4; 125 mM NaCl; 24 mM MgCl₂; 100 *µ*M sodium orthovanadate), in a total volume of 10 *µ*l, for 20-30 minutes at room temperature. The test compound, dissolved in dimethylsulfoxide (DMSO), is diluted in PBV, and 10 *µ*l is mixed with the EGF receptor /EGF mix, and incubated for 10-30 minutes at 30°C. The phosphorylation reaction is initiated by addition of 20 *µ*l ³³P-ATP/ substrate mix (120*µ*M Lys₃-Gastrin (sequence in single letter code for amino acids, KKKGPWLEEEEEAYGWLDF), 50 mM Hepes pH 7.4, 40 *µ*M ATP, 2 *µ*Ci *γ*-[³³P]-ATP) to the EGFr/EGF mix and incubated for 20 minutes at room temperature. The reaction is stopped by addition of 10 *µ*l stop solution (0.5 M EDTA, pH 8; 2mM ATP) and 6 *µ*l 2N HCl. The tubes are centrifuged at 14,000 RPM, 4°C, for 10 minutes. 35 *µ*l of supernatant from each tube is pipetted onto a 2.5 cm circle of Whatman P81 paper, bulk washed four times in 6% acetic acid, 1 liter per wash, and then air dried. This results in the binding of substrate to the paper with loss of free ATP on washing. The [³³P] incorporated is measured by liquid scintillation counting. Incorporation in the absence of substrate (e.g., lys₃-gastrin) is subtracted from all values as a background and percent inhibition is calculated relative to controls without test compound present.

Such assays, carried out with a range of doses of test compounds, allow the determination of an approximate IC₅₀ value for the in vitro inhibition of EGFR kinase activity. Although the inhibitory properties of the compound of Formula I vary with structural change as expected, the activity generally exhibited by this agent, determined in the manner described above, is in the range of IC₅₀=0.0001-30 *µ*M.

Activity of the active compound , in vivo, can be determined by the amount of inhibition of tumor growth by a test compound relative to a control. The tumor growth inhibitory effects of various compounds are measured according to the methods of Corbett T. H., et al. "Tumor Induction Relationships in Development of Transplantable Cancers of the Colon in Mice for Chemotherapy Assays, with a Note on Carcinogen Structure', Cancer Res., 35, 2434-2439 (1975) and Corbett, T. H., et al., "A Mouse Colon-tumor Model for Experimental Therapy", Cancer Chemother. Rep. (Part 2)", 5, 169-186 (1975), with slight modifications. Tumors are Induced in the left flank by s.c. Injection of 1 X 10⁶ log phase cultured tumor cells (human MDA-MB-468 breast or human HN5 head and neck carcinoma cells) suspended in 0.10 ml RPMI 1640. After sufficient time has elapsed for the tumors to become palpable (2-3 mm in diameter) the test animals (athymic mice) are treated with active compound (formulated by dissolution in DMSO typically at a concentration of 50 to 100 mg/mL followed by 1:9 dilution into saline or, alternatively, 1:9 dilution into 0.1% Pluronic" P105 in 0.9% saline) by the intraperitoneal (ip) or oral (po) routes of administration twice daily (i.e., every 12 hours) for 5 consecutive days. In order to determine an anti-tumor effect, the tumor is measured in millimeters with Vernier calipers across two diameters and the tumor size (mg) is calculated using the formula: Tumor weight = (length x [width]²)/2, according to the methods of Geran, R.I., et al. "Protocols for Screening Chemical Agents and Natural Products Against Animal Tumors and Other Biological Systems", Third Edition, Cancer Chemother. Rep., 3, 1-104 (1972).. Results are expressed as percent inhibition, according to the formula: Inhibition (%) = (TuW_{control} - TuWₜₑₛₜ/TuW_{control} x 100%. The flank site of tumor implantation provides reproducible dose/response effects for a variety of chemotherapeutic agents, and the method of measurement (tumor diameter) is a reliable method for assessing tumor growth rates.

Administration of the active compound can be effected by any method which enables delivery of the compounds to the site of action (e.g., cancer cells). These methods include oral routes, intraduodenal routes, parenteral injection (including intravenous, subcutaneous, intramuscular, intravascular or infusion), topical administration, etc.

The amount of active compound administered will, of course, be dependent on the subject being treated, on the severity of the affliction, on the manner of administration and on the judgement of the prescribing physician. However an effective dosage is In the range of approximately 0.001-100 mg/kg, preferably 1 to 35 mg/kg in single or divided doses. For an average 70kg human, this would amount to 0.05 to 7 g/day, preferably 0.2 to 2.5 g/day.

The composition may, for example, be in a form suitable for oral administration as a tablet, capsule, pill, powder, sustained release formulations, solution, suspension, for parenteral injection as a sterile solution, suspension or emulsion, for topical administration as an ointment or cream or for rectal administration as a suppository. The pharmaceutical composition may be In unit dosage forms suitable for single administration of precise dosages. The pharmaceutical composition will include a conventional pharmaceutical carrier or excipient and the compound according to the invention as an active ingredient. In addition, it may include other medicinal or pharmaceutical agents, carriers, adjuvants, etc.

Pharmaceutical compositions according to the invention may contain 0.1%-95% of the compound, preferably 1%-70%. In any event, the composition or formulation to be administered will contain a quantity of active compound In an amount effective to alleviate or reduce the signs in the subject being treated, i.e., hyperproliferative diseases, over the course of the treatment.

Exemplary parenteral administration forms include solutions or suspensions of active compounds in sterile aqueous solutions, for example aqueous propylene glycol or dextrose solutions. Such dosage forms can be suitably buffered, if desired.

Suitable pharmaceutical carriers include inert diluents or fillers, water and various organic solvents. The pharmaceutical compositions may, if desired, contain additional ingredients such as flavorings, binders, excipients and the like. Thus for oral administration, tablets containing various excipients, such as citric acid may be employed together with various disintegrants such as starch, alginic acid and certain complex silicates and with binding agents such as sucrose, gelatin and acacia. Additionally, lubricating agents such as magnesium stearate, sodium lauryl sulfate and talc are often useful for tableting purposes. Solid compositions of a similar type may also be employed in soft and hard filled gelatin capsules. Preferred materials, therefor, include lactose or milk sugar and high molecular weight polyethylene glycols. When aqueous suspensions or elixirs are desired for oral administration the active compound therein may be combined with various sweetening or flavoring agents, coloring matters or dyes and, If desired, emulsifying agents or suspending agents, together with diluents such as water, ethanol, propylene glycol, glycerin, or combinations thereof.

Methods of preparing various pharmaceutical compositions with a specific amount of active compound are known, or will be apparent, to those skilled in this art. For examples, see Remington's Pharmaceutical Sciences., Mack Publishing Company, Easter, Pa., 15th Edition (1976).

The hyperproliferative disease treatment described above may be applied as a sole therapy or may involve, in addition to the active compound, one or more other antitumor substances. Such conjoint treatment may be achieved by way of the simultaneous, sequential, cyclic or separate dosing of the individual components of the treatment.

High pressure liquid chromatography (HPLC) used In the following examples and preparations was effected according to the following method unless modified in specific examples. Perkin-Elmer Pecosphere" 3X3C cartridge column (3mm X 3cm, C18; available from Perkin Elmer Corp., Norwalk, CT 06859) with a Brownlee (trademark) RP-8 Newguard precolumn (7 micron, 3.2mm X 15mm, available from Applied Biosystems Inc. San Jose, CA 95134) which was previously equilibrated in pH 4.50, 200 mM ammonium acetate buffer. Samples were eluted using a linear gradient of 0-100% acetonitrile/pH4.50, 200 mM NH₄ acetate over 10 minutes with a flow rate of 3.0 mL/min. Chromatograms were generated over the range 240-400nm using a diode array detector.

### EXAMPLE 1

### [6-,7-Bis-(2-methoxyethoxy)-quinazolin-4-yl]-(3-ethynylphenyl)amine hydrochloride

3-Ethynylaniline (37 mg, 0.32 mmol.), and 4-chloro-6,7-bis-(2-methoxy-ethoxy)-quinazoline (90 mg, 0.29 mmol) were added to isopropanol (1.5 mL) containing pyridine (25 *µ*L, 0.32 mmol) and the mixture was refluxed 4 hours under an atomospher of dry nitrogen. The solvent was removed, in vacuo, and the residue partitioned between 10% methanol in CHCl₃ and saturated aqueous NaHCO₃. The organic phase was dried over Na₂SO₄, filtered and concentrated in vacuo. The residue was flash chromatographed on silica using 30% acetone in hexanes to afford 81 mg of the free base of the title product as a pale yellow solid. The free-base was dissolved in a minimum volume of CHCl₃, diluted with several volumes of ether, and titrated with 1M HCl in ether to precipitate the title product as its hydrochloride salt; 90 mg; 71%; mp 228-230 °C.

### PREPARATION 1

### 6,7-Bis(2-methoxy-ethoxy)-quinazolone

To ethyl 3,4-dihydroxybenzoate (36.4 g, 0.200 mol), K₂CO₃ (60.8 g, 0.44 mol) and tetrabutylammonium iodide (750 mg) in degassed acetone (400 mL) was added 2-bromoethyl methyl ether (69.5 g, 47 mL). The mixture was stirred under N₂ at reflux for 64 hours. Ether (600 mL) was added to the mixture and after stirring 30 minutes at 20 °C the precipitated salts were removed by filtration. The filtrate was concentrated in vacuo and the residue was triturated with hexane (500 mL) for 30 minutes and the white solid ethyl 3,4-bis(2-methoxy-ethoxy)benzoate was filtered and dried in vacuo (55.5 g; 93%; M.P. 50-51 °C). A portion of this product (45,7 g, 0.158 mol) in acetic acid (150 mL) was treated dropwise with cone. HNO₃ (40 mL) at 5°C and the solution stirred 24 hours before pouring into cold H₂O (1.6 L). The mixture was extracted with ethyl acetate (1.1 L), and the organic phase was washed three times with 200 mL H₂O, and brine, dried over Na₂SO₄, filtered and concentrated in vacuo to afford ethyl 4,5-bis-(2-methoxy-ethoxy)-2-nitro-benzoate (54.3 g) as a brown oil. This nitro product (52.0 g, 0.15 mol) was dissolved in ethanol (1000 mL) containing 1 equivalent of HCl (generated in the ethanol by prior addition of 11 mL acetyl chloride), PtO₂●H₂O (1.0 g) was added, and the mixture was hydrogenated under 45 psi H₂ for 6 hours. The catalyst was removed by filtration through Celite, and the filtrate was concentrated in vacuo to a thick slurry which was diluted with ether (400 mL). The solid white hydrochloride salt of ethyl 2-amino-4,5-bis-(2-methoxy-ethoxy)benzoate was filtered and dried in vacuo (44.7 g; 88%). A portion of this material (42 g, 0.12 mol) and ammonium formate (7.6 g, 0.12 mol) were disssolved in formamide (63 mL) and the stirred mixture was heated to 160-165 °C under an atmosphere of N₂ for 3 hours. H₂O (200 mL) was added and after cooling the precipitated crude title product was recovered by filtration, washed with cold H₂O, and dried in vacuo. The filtrate was extracted five times with CHCl₃, and the pooled organic extracts were washed with brine, dried over Na₂SO₄, and concentrated in vacuo. The residue and crude quinazolone precipitate were combined, triturated in hot acetonitrile (250 mL) for 30 minutes, cooled to 20 °C and treated with ether (250 mL). After cooling to 4 °C the white solid was filtered and dried in vacuo (30.4 g, 86%; GC-MS m/z 294 (M⁺)).

### PREPARATION 2

### 4-Chloro-6,7-bis-(2-methoxy-ethoxy)-quinazoline

To 6,7-bis(2-mothoxy-othoxy)-quinazolone (500 mg, 1.7 mmol), from Preparation 1, in CHCl₃ (10 mL) containing one drop of DMF was added oxalylchloride (490*µ*L, 5.6 mmol) in several portions over 5 minutes. Once foaming ceased the solution was refluxed 1.5 hours. The solvent was removed In vacuo and the residue was dissolved in 1,2-dichloroethane (20 mL) and washed two times with 80 mL saturated aqueous Na₃CO₃. The organic phase was dried over Na₂SO₄, and concentrated in vacuo to afford solid title product (520 mg, 92%; M.P. 108-109 °C).

### PREPARATION 3

### 4-chloro-6,7-bis-(2-methoxy-ethoxy)-quinazoline

6,7-Bis(2-mothoxy-ethoxy)-quinazolone (5.4 g, 18.3 mmol), from Preparation 1, and pyridine (3.0 mL, 37 mmol) were heated in refluxing POCl₃ (22 mL) under an atmosphere of dry nitrogen for 2.5 hours. Following concentration of the mixture in vacuo at 60°C the residue was dissolved in CHCl₃ (150 mL) and carefully added in portions with stirring to cold saturated aqueous NaHCO₃ (100 mL). The mixture was stirred 10 min. after the addition was complete and the organic phase was separated, washed with brine, dried over Na₂SO₄, and concentrated in vacuo. The residue was flash chromatographed on silica using a gradient of 20% to 60% ethyl acetate/hexanes to afford 3.41 g of 4-chloro-6,7-bis-(2-methoxy-ethoxy)-quinazoline.

## Claims

1. A composition for use as a medicament for treating hyperproliferative diseases comprising a combination of [6,7-Bis-(2-methoxyethoxy)quinazolin-4-yl]-(3-ethynylphenyl)amine, or a pharmaceutically acceptable salt thereof, and one or more additional antitumor substances.

2. [6,7-Bis-(2-methoxyethoxy)quinazolin-4-yl]-(3-ethynylphenyl)amine, or a pharmaceutically acceptable salt thereof, in combination with one or more additional antitumor substances for treating hyperproliferative diseases.

3. A pharmaceutical combination comprising:
[6,7-Bis-(2-methoxyethoxy)quinazoline-4-yl]-(3-ethynylphenyl)amine, or a pharmaceutically acceptable salt thereof; and one or more additional antitumor substances.

## Patentansprüche

1. Zusammensetzung zur Verwendung als Medikament zur Behandlung von hyperproliferativen Erkrankungen, mit einer Kombination aus [6,7-Bis-(2-Methoxyethoxy)chinazolin-4-yl]-(3-Ethynylphenyl)amin, oder einem pharmazeutisch zulässigen Salz desselben, und einer oder mehreren zusätzlichen Antitumorsubstanzen.

2. [6,7-Bis-(2-Methoxyethoxy)chinazolin-4-yl]-(3-Ethynylphenyl)amin, oder ein pharmazeutisch zulässiges Salz desselben, in Kombination mit einer oder mehreren zusätzlichen Antitumorsubstanzen zur Behandlung von hyperproliferativen Erkrankungen.

3. Pharmazeutische Kombination mit:
[6,7-Bis-(2-Methoxyethoxy)chinazolin-4-yl]-(3-Ethynylphenyl)amin, oder einem pharmazeutisch zulässigen Salz desselben; und einer oder mehreren zusätzlichen Antitumorsubstanzen.

## Revendications

1. Composition pour une utilisation en tant que médicament destiné au traitement de maladies hyperprolifératives, comprenant une combinaison de [6,7-bis(2-méthoxyéthoxy)quinazolin-4-yl]-(3-éthynylphényl)-amine, ou d'un sel pharmaceutiquement acceptable de celle-ci, et d'une ou plusieurs substance(s) antitumorale(s) additionnelle(s).

2. [6,7-Bis(2-méthoxyéthoxy)quinazolin-4-yl]-(3-éthynylphényl)amine, ou un sel pharmaceutiquement acceptable de celle-ci, en combinaison avec une ou plusieurs substance(s) antitumorale(s) additionnelle(s), pour le traitement de maladies hyperprolifératives.

3. Combinaison pharmaceutique comprenant de la [6,7-bis(2-méthoxyéthoxy)quinazolin-4-yl]-(3-éthynyl-phényl)amine, ou un sel pharmaceutiquement acceptable de celle-ci, et d'une ou plusieurs substance(s) antitumorale(s) additionnelle(s).
